# EUROPÄISCHE PATENTSCHRIFT

(11) **EP 2 529 392 B1**
(45) Veröffentlichungstag und Bekanntmachung des Hinweises auf die Patenterteilung: **02.04.2014**
(21) Anmeldenummer: 11778839.8
(22) Anmeldetag: 26.10.2011
(51) Int. Cl.: H01J 65/04, H01J 5/48, H01J 61/30, A61L 2/10, H01J 5/56

(54) **STRAHLER MIT SOCKEL FÜR DIE BESTRAHLUNG VON OBERFLÄCHEN**
RADIATING ELEMENT FOR IRRADIATING SURFACES, HAVING A SOCKET
ÉMETTEUR DE RAYONNEMENT DOTÉ D'UN SOCLE POUR EXPOSER DES SURFACES À UN RAYONNEMENT

(30) Priorität: 02.11.2010 DE 102010043215
(43) Veröffentlichungstag der Anmeldung: 05.12.2012
(73) Patentinhaber: OSRAM GmbH, 80807 München (DE)
(72) Erfinder: HALFMANN, Helmut, 51688 Wipperfuerth (DE); HOMBACH, Axel, 51515 Kürten (DE); ROTH, Markus, 53129 Bonn (DE)
(86) Internationale Anmeldenummer: PCT/EP2011/068766
(87) Internationale Veröffentlichungsnummer: WO 2012/059382

(56) Entgegenhaltungen:
- EP-A1- 0 489 184
- EP-A2- 1 601 003
- US-A- 5 013 959
- US-A1- 2008 265 775
- US-B1- 6 379 024

## Beschreibung

### Technisches Gebiet

Die Erfindung geht aus von einem Strahler mit einem Sockel für die Bestrahlung von Oberflächen mit elektromagnetischer Strahlung. Insbesondere betrifft die Erfindung einen Strahler, der kurzwellige Ultraviolett(UV)-Strahlung, vorzugsweise unterhalb von 200 nm, abstrahlt. Derartige Strahler können unter anderem für die Entkeimung von Oberflächen, beispielsweise ebenen Folien sowie das Innere von Flaschen, Kanistern oder anderen hohlen Behältern, benutzt werden.

### Stand der Technik

Bei der UV-Bestrahlung von Oberflächen, insbesondere der Entkeimung, kann es vorteilhaft sein, wenn zumindest während der Bestrahlung, gegebenenfalls auch davor und/oder danach, eine Spülung des Raumbereichs zwischen Strahler und Oberfläche mit einem Spülgas stattfindet. Dadurch sollen zumindest während der Bestrahlung UV-Strahlung absorbierende Gase zwischen der zu bestrahlenden Oberfläche und dem UV-Strahler entfernt werden. Als Spülgas eignen sich insbesondere Stickstoff oder Edelgase. Auch bei anderen Bestrahlungsprozessen kann eine definierte Atmosphäre zwischen Strahler und Oberfläche und folglich die Verwendung eines geeigneten Prozessgases vorteilhaft sein. Im Folgenden wird der Begriff Prozessgas als Oberbegriff für ein oder mehrere geeignete Gase zum Spülen oder für sonstige Prozesse vor, während und/oder nach der Bestrahlung verwendet.

Für die Bestrahlung gut zugänglicher Oberflächen, beispielsweise ebener Folien, wird das Spülgas bisher mittels einer separaten Vorrichtung um den Strahler herum bzw. von der Seite zugeführt. Nachteilig ist der zusätzliche apparative Aufwand und die erforderliche Abstimmung zwischen Spülanordnung und Strahler. Außerdem lassen sich diese Systeme aus Platzgründen in der Regel nicht innerhalb von Hohlräumen mit engen Zugangsöffnungen wie Kanister oder Flaschen einsetzen.

Aus der Schrift EP 1 506 567 B1 ist ein UV-Strahler auf der Basis einer einseitigen dielektrischen Barriere-Entladung bekannt. Dazu ist das Entladungsgefäß 2 mit Xenon gefüllt. Während der Gasentladung, die bevorzugt mittels eines in der US 5 604 410 beschriebenen gepulsten Betriebsverfahrens betrieben wird, werden sogenannte Excimere gebildet. Excimere sind angeregte Moleküle, z.B. Xe2*, die bei der Rückkehr in den in der Regel ungebundenen Grundzustand elektromagnetische Strahlung emittieren. Im Falle von Xe2* liegt das Maximum der Molekülbandenstrahlung bei ca. 172 nm. Zur Erzeugung der dielektrischen Barriere-Entladung ist eine erste wendelförmige Elektrode 23 koaxial innerhalb des rohrförmigen Entladungsgefäßes 2 angeordnet. Auf der Außenseite des Entladungsgefäßes 2 sind sechs streifenförmige Außenelektroden 8a-8f parallel zueinander und mit gegenseitigem Abstand angeordnet.

In der Schrift EP 0 607 960 A1 ist ein rohrförmiger UV-Strahler auf der Basis einer zweiseitigen dielektrischen Barriere-Entladung offenbart. Das Strahlergefäß ist in der Art einer koaxialen Doppelrohranordnung gebildet, bei der ein Innenrohr und ein Außenrohr an den beiden Stirnseiten gasdicht miteinander verbunden sind. Der vom Entladungsgefäß umschlossene Entladungsraum erstreckt sich bei dieser Anordnung zwischen Innen- und Außenrohr.

In der Schrift EP 1 232 518 B1 ist eine flache Entladungslampe auf der Basis einer zweiseitigen dielektrischen Barrieren-Entladung offenbart. Die dielektrische Barrieren-Entladung wird zwischen einer Boden- und einer Deckenplatte erzeugt, wobei der umlaufende Dichtungsrahmen und trichterförmige Stützelemente in die Deckenplatte eingeformt sind. Die Elektroden sind als zwei ineinander greifende kammartige Linienstrukturen auf die Außenseite der Bodenplatte aufgebracht.

Aus der Schrift US 5013959 A ist ein flacher Strahler zur Bestrahlung von Oberflächen bekannt, der eine Vielzahl rohrförmiger Entladungslampen umfasst.

### Darstellung der Erfindung

Die Aufgabe der vorliegenden Erfindung ist es, einen Strahler bereit zu stellen, der eine Bestrahlung von Oberflächen unter definierter Atmosphäre ermöglicht.

Diese Aufgabe wird gelöst durch einen Strahler für die Bestrahlung von Oberflächen gemäß Anspruch 1.

Besonders vorteilhafte Ausgestaltungen finden sich in den abhängigen Ansprüchen.

Außerdem wird Schutz beansprucht für die Verwendung des erfindungsgemäßen Strahlers für die Entkeimung von ebenen oder gekrümmten Oberflächen.

Der Grundgedanke der Erfindung besteht darin, die Gasspülung mit einem Prozessgas, beispielsweise einem Inertgas wie Stickstoff oder einem Edelgas, nicht mit Hilfe einer separaten Vorrichtung auszuführen, sondern einen Strahler geeignet so weiterzubilden, dass ein Prozess/Spülgas aus dem Strahler austreten kann und damit dem Raumbereich zwischen Strahler und zu bestrahlender Oberfläche zugeführt wird. Dadurch verringert sich nicht nur der apparative sondern auch zeitliche Aufwand, insbesondere bei der Bestrahlung der Innenflächen von Hohlkörper, wo bisher in der Regel ein Gasaustausch vor der Bestrahlung nötig ist. Außerdem verringert sich der Platzbedarf für die Vorrichtung.

Zu diesem Zweck ist der erfindungsgemäße Strahler so ausgebildet, dass das Prozessgas aus dem Strahlersockel selbst ausströmt und auf diese Weise dem benachbarten Raumbereich der zu bestrahlenden Oberfläche zugeführt werden kann. Dazu weist der Strahlersockel Gasöffnungen auf, aus der das Prozessgas ausströmen kann. Vorzugsweise sind die Gasöffnungen am Strahlersockel so angeordnet bzw. ausgerichtet, dass die Gasströmung in Richtung Strahlergefäß bzw. in den dazu benachbarten Raumbereich strömen kann.

Zusätzlich kann vorgesehen sein, dass das Prozessgas auch durch das Strahlergefäß hindurchströmt und damit ebenfalls dem Raumbereich zwischen Strahler und zu bestrahlender Oberfläche zugeführt wird. Dabei tritt das Prozessgas selbstverständlich nicht durch den Entladungsraum selbst, in dem im Betrieb eine Gasentladung die Strahlung erzeugt. Vielmehr ist der Entladungsraum auch in diesem Fall hermetisch durch das Entladungsgefäß abgeschlossen. Das Prozessgas strömt nämlich gegebenenfalls durch eine oder mehrere tunnelartige Durchgangsöffnungen im Entladungsgefäß. Dadurch kann das Prozessgas ohne Einfluss auf die innerhalb des hermetisch abgeschlossenen Entladungsgefäßes erzeugte Gasentladung durch die tunnelartigen Durchlassöffnungen hindurchströmen. Dabei kann auch vorgesehen sein, dass - zumindest zeitweise - von Gaszufuhr auf Gasabfuhr umgeschaltet werden kann.

Zusätzlich kann auch eine oder mehrere Gasöffnungen oder tunnelartige Durchgangsöffnungen generell für die Gasabfuhr vorgesehen sein. Das ist insbesondere für die Entkeimung von hohlen Behältern wie Flaschen oder Kanister mit engen Behälteröffnungen vorteilhaft, wo unter Umständen wenig Platz für das Abströmen des Prozessgases verbleibt. In einer Weiterbildung des erfindungsgemäßen Strahlers ist außerdem eine Dichtung zwischen Strahler und Behälteröffnung vorgesehen, um das Eindringen von Umgebungsluft in den Behälter zu verhindern. Hier erfolgt das Abströmen des Prozessgases ausschließlich über die hierfür vorgesehene(n) Gasöffnung(en) bzw. Durchgangsöffnung(en) im Strahler.

Zum Versorgen mit Prozessgas ist der Strahler vorzugsweise mit einem Anschluss versehen, an den eine Gasleitung angeschlossen werden kann. Alternativ kann der Strahler auch unmittelbar mit einer Gasleitung verbunden sein.

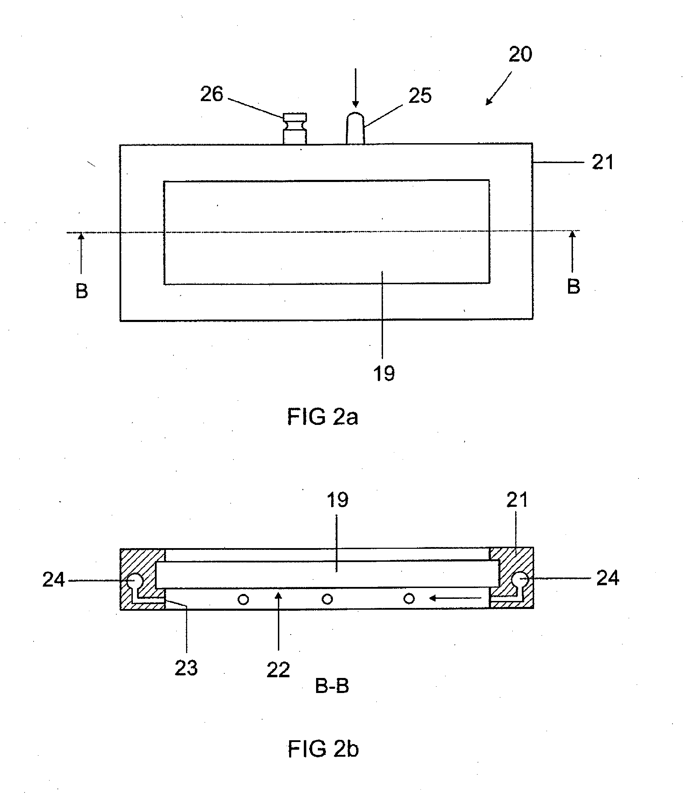

Der Strahler ist generell für die Bestrahlung von ausgedehnten, beispielsweise planen Flächen, beispielsweise Folienbahnen, geeignet. Dafür eignet sich besonders erfindungsgemäße Ausführungsform, wobei der Strahler und insbesondere das Strahlergefäß eine flache Form aufweisen. Mit anderen Worten basiert die Erfindung auf einer so genannten Flachlampe, die gegebenenfalls für die gewünschte Strahlung, insbesondere UV-Strahlung für die Entkeimung, modifiziert ist. Hierbei weist der rahmenförmige Strahlersockel mehrere Gasöffnungen auf, aus der die Gasströmung austreten kann. Zusätzlich kann auch das flache Strahlergefäß selbst mit tunnelartigen Durchgangsöffnungen für das Prozessgas versehen sein. Für weitere Details sei auf das in den Figuren 2a und 2b gezeigte Ausführungsbeispiel verwiesen.

Generell besteht das Strahlergefäß zumindest abschnittsweise aus einem für die Strahlung transparenten Material, im Falle von UV-Strahlung vorzugsweise Quarzglas.

Für die Erzeugung von UV-Strahlung ist der Strahler vorzugsweise für eine dielektrische Barrieren-Entladung im Inneren des Strahlergefäßes ausgelegt. Für weitere Details hierzu sei auf die eingangs erwähnten Schriften EP 1 506 567 B1, US 5 604 410 und EP 1 232 518 B1 verwiesen.

### Kurze Beschreibung der Zeichnungen

Im Folgenden soll die Erfindung anhand von Ausführungsbeispielen näher erläutert werden. Die Figuren zeigen:
- Fig. 1a: eine Längsteilschnittsdarstellung eines rohrförmigen Strahlers,
- Fig. 1b: eine Querschnittsdarstellung des Strahlers aus Fig. 1a,
- Fig. 2a: eine Draufsicht auf einen erfindungsgemäßen flachen Strahler,
- Fig. 2b: eine Längsteilschnittdarstellung des Strahlers aus Fig. 2a.

### Bevorzugte Ausführung der Erfindung

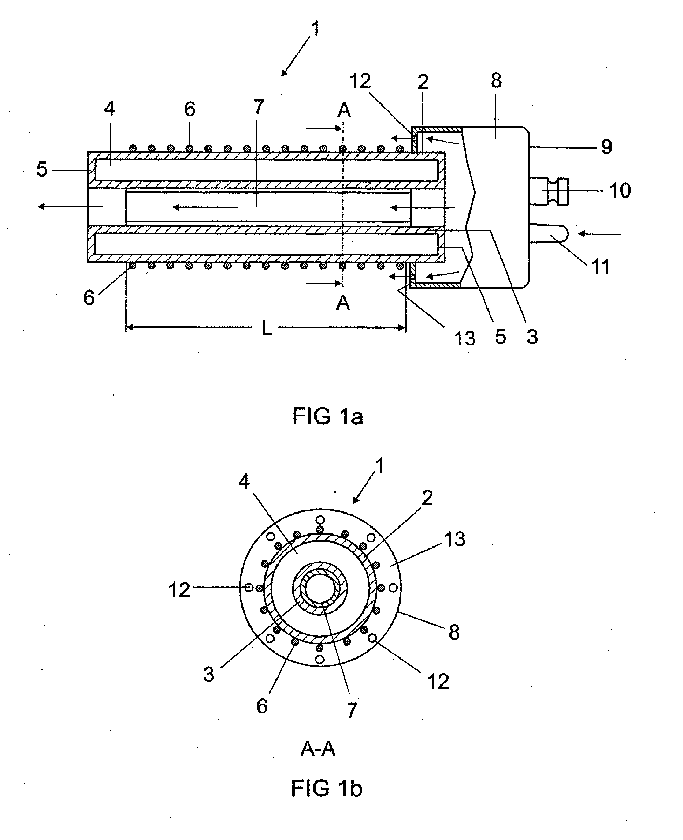

Die Figuren 1a, 1b zeigen in stark schematisierter Darstellung eine teilweise geschnittene Längsansicht bzw. eine Querschnittsansicht eines Beispiels eines Strahlers 1 auf der Basis einer dielelektrischen Barrieren-Entladung. Das längliche Entladungsgefäß des Strahlers 1 besteht aus einem Außenrohr 2 und einem Innenrohr 3 in koaxialer Doppelrohranordnung, die so die Längsachse des Entladungsgefäßes definieren. Die Länge der Rohre variiert je nach Anwendung. Für die Entkeimung von Flaschen beispielsweise ist die Länge vorzugsweise so dimensioniert, dass die Flascheninnenfläche bei eingetauchtem Strahler komplett bestrahlt wird. Die Durchmesser der Rohre sind ebenfalls vorzugsweise an die Anwendung angepasst. Insbesondere ist der größte Außendurchmesser des Entladungsgefäßes so bemessen, dass der Strahler 1 in den für die Bestrahlung vorgesehenen Behälter, beispielsweise durch den Flaschenhals hindurch in eine Flasche, eingeführt werden kann. Beide Rohre 2, 3 bestehen aus UV-Strahlung durchlässigem Quarzglas. Außerdem ist das Entladungsgefäß an seinen beiden Stirnseiten derart verschlossen, dass ein länglicher, ringspaltförmiger Entladungsraum 4 gebildet ist. Zu diesem Zweck weist das Entladungsgefäß an seinen beiden Enden jeweils geeignet geformte, ringartige Gefäßabschnitte 5 auf. Außerdem ist an einem der Gefäßabschnitte 5 ein Pumprohr (nicht dargestellt) angesetzt, mit Hilfe dessen der Entladungsraum 4 zunächst evakuiert und anschließend mit 15 kPa Xenon gefüllt wird. Auf der Außenseite der Wand des Außenrohrs 2 ist ein Drahtnetz 6 aufgezogen, das die Außenelektrode der Lampe 1 bildet. Alternativ kann hierfür beispielsweise auch eine schmale spiralförmige Metallbahn aufgetragen werden. Im Inneren des Innenrohrs 3, d.h. ebenfalls außerhalb des durch das Entladungsgefäß umschlossenen Entladungsraums 4, ist ein Metallrohr 7 angeordnet, das die Innenelektrode der Lampe bildet. Alternativ kann hierfür beispielsweise auch eine leitende Schicht, z.B. aus Kohlenstoff, aufgetragen werden. An einem Ende des Entladungsgefäßes ist ein topfförmiger Sockel 8 angeordnet. Die vom Entladungsgefäß abgewandte Stirnseite 9 weist eine elektrische Buchse 10 zum Anschluss der Versorgungsspannung für den Strahler 1 auf. Außerdem ist auf der Stirnseite 9 ein Gasstutzen 11 angebracht, an den ein Prozessgasschlauch aufgesteckt werden kann. Das über den Gasstutzen 11 einströmende Prozessgas gelangt im Inneren des Sockels 8 zu insgesamt acht Löchern 12, die in dem ringförmigen Sockelvorsprung 13, aus dem das Entladungsgefäß herausragt, im Umfang gleichmäßig verteilt angeordnet sind. Dadurch kann das Prozessgas aus diesen Löchern 12 austreten und entlang des Außenrohrs 2 dessen benachbartem Raumbereich zuströmen. Außerdem ist der Sockel 8 im Innern so ausgebildet, dass das Prozessgas zum sockelseitigen Ende des Innenrohrs 3 einströmen und an dessen anderem Ende ausströmen kann. Das Innenrohr fungiert also für das Prozessgas zusätzlich als tunnelartige Durchgangsöffnung durch das Entladungsgefäß hindurch. Alternativ kann das Innenrohr auch für die Abfuhr des Prozessgases vorgesehen sein und/oder ein Teil der Gasöffnungen im Sockel.

Die Figuren 2a, 2b zeigen in stark schematisierter Darstellung eine Draufsicht und einen Teillängsschnitt auf einen erfindungsgemäßen flachen Strahler 20. Dieser weist ein flaches Strahlergefäß 19 und einen Strahlersockel 21 auf, der das flache Strahlergefäß 19 rahmenförmig einfasst. Beim Strahlergefäß 19 handelt es sich um eine modifizierte Flachlampe (nicht geschnitten dargestellt) auf der Basis einer dielelektrischen Barrieren-Entladung wie sie beispielsweise aus der eingangs erwähnten EP 1 232 518 B1 bekannt ist. Für die Entkeimung mittels UV-Strahlung wird auf den ansonsten üblichen Leuchtstoff verzichtet. Außerdem besteht das flache Entladungsgefäß aus Quarzglas wegen der erforderlichen Transparenz für UV-Strahlung. Das Entladungsgefäß selbst hat eine rechteckige Grundform. Die Elektroden des Strahlergefäßes sind wie bei diesen Lampentypen bekannt ausgebildet und der Übersicht wegen hier nicht dargestellt. Der Strahlersockel 21 ragt insbesondere über die die Strahlung abstrahlende Vorderseite 22 des Strahlergefäßes hinaus. Dort sind auf den vorstehenden Innenseiten des rahmenförmigen Strahlersockels 21 mehreren Gasöffnungen 23 so angeordnet, dass das Prozessgas von seitlich über die Vorderseite 22 des Flachstrahlers und folglich auch über die während der Bestrahlung unmittelbar benachbarte zu bestrahlende Oberfläche strömen kann. Die insgesamt acht Gasöffnungen 23 (2 mal 1 für beide Schmalseiten plus 2 mal 3 für die beiden Längsseiten) werden über eine Ringgasleitung 24 gespeist, die wiederum mit einem am Strahlersockel 21 angeordneten Gasanschlussstutzen 25 verbunden ist. Außerdem weist der Strahlersockel 21 eine elektrische Buchse 26 zum Anschluss der Versorgungsspannung für den Betrieb des Flachstrahlers auf. In einer nicht dargestellten Variante ist das Flachstrahlergefäß mit tunnelartigen Durchgangsöffnungen versehen, die einen zusätzlichen Gasstrom durch das Flachstrahlergefäß, d.h. im wesentlichen senkrecht zu der zu bestrahlenden Oberfläche ermöglichen. Die tunnelartigen Durchgangsöffnungen können beispielsweise bei einer Flachlampe der in der eingangs erwähnten EP 1 232 518 B1 offenbarten Art durch gasdichtes Durchstoßen einer oder mehrerer der aus den flachen Gefäßteilen geformten trichterartigen Stützstellen erfolgen. Die tunnelartigen Durchgangsöffnungen im Flachstrahlergefäß können alternativ auch zur Abfuhr des Prozessgases vorgesehen sein.

## Patentansprüche

1. Strahler (20) für die Bestrahlung von Oberflächen, mit einem Strahlergefäß (19) und einem damit verbundenen Strahlersockel (21), wobei der Strahlersockel (21) mindestens eine Gasöffnung (23) aufweist, die für die Zufuhr eines Prozessgases in einen dem Strahlergefäß (19) benachbarten Raumbereich ausgebildet ist, **dadurch gekennzeichnet, dass** das Strahlergefäß (19) flach und der Strahlersockel (21) rahmenförmig ausgebildet ist, wobei der Strahlersockel (21) über die im Betrieb Strahlung abstrahlende Vorderseite (22) des Strahlergefäßes (19) hinaus ragt und auf den vorstehenden Innenseiten des rahmenförmigen Strahlersockels (21) mehrere Gasöffnungen (23) angeordnet sind.

2. Strahler nach Anspruch 1, wobei die Gasöffnungen (23) so angeordnet und orientiert sind, dass das Prozessgas in einen dem Strahlergefäß (19) benachbarten Raumbereich hineinströmen kann.

3. Strahler nach Anspruch 1 oder 2, wobei das Strahlergefäß mit mindestens einer tunnelartigen Durchgangsöffnung versehen ist, die für die Zu- oder Abfuhr des Prozessgases vorgesehen ist.

4. Strahler nach einem der vorstehenden Ansprüche mit einem Gasanschluss (25) für die Versorgung mit Prozessgas.

5. Strahler nach einem der vorstehenden Ansprüche, wobei der Strahlersockel mindestens eine Gasöffnung aufweist, die für die Abfuhr eines Prozessgases ausgebildet ist.

6. Strahler nach einem der vorstehenden Ansprüche, wobei das Strahlergefäß (19) zumindest abschnittsweise aus einem für die Strahlung transparenten Material besteht.

7. Strahler nach einem der vorstehenden Ansprüche, der für die Emission von UV-Strahlung ausgelegt ist.

8. Strahler nach einem der vorstehenden Ansprüche, der für eine dielektrische Barrieren-Entladung im Inneren des Strahlergefäßes (19) ausgelegt ist.

9. Verwendung eines Strahlers gemäß einem der vorstehenden Ansprüche für die Entkeimung von ebenen oder gekrümmten Oberflächen.

## Claims

1. Emitter (20) for the irradiation of surfaces, comprising an emitter vessel (19) and an emitter base (21) connected thereto, wherein the emitter base (21) has at least one gas opening (23), which is designed for supplying a process gas into a spatial area adjacent to the emitter vessel (19), **characterized in that** the emitter vessel (19) is flat, and the emitter base (21) is in the form of a frame, wherein the emitter base (21) protrudes beyond the front side (22) of the emitter vessel (19), which front side emits radiation during operation, and a plurality of gas openings (23) are arranged on the protruding inner sides of the frame-shaped emitter base (21).

2. Emitter according to Claim 1, wherein the gas openings (23) are arranged and oriented in such a way that the process gas can flow into a spatial area adjacent to the emitter vessel (19).

3. Emitter according to Claim 1 or 2, wherein the emitter vessel is provided with at least one tunnel-like through-opening, which is provided for supplying or discharging the process gas.

4. Emitter according to one of the preceding claims, comprising a gas connection (25) for the supply of process gas.

5. Emitter according to one of the preceding claims, wherein the emitter base has at least one gas opening, which is designed for the discharge of a process gas.

6. Emitter according to one of the preceding claims, wherein the emitter vessel (19) consists at least sectionally of a material which is transparent to the radiation.

7. Emitter according to one of the preceding claims, which is designed for the emission of UV radiation.

8. Emitter according to one of the preceding claims, which is designed for a dielectric barrier discharge in the interior of the emitter vessel (19).

9. Use of an emitter according to one of the preceding claims for sterilizing flat or curved surfaces.

## Revendications

1. Émetteur (20) pour l'exposition de surfaces à un rayonnement, comprenant une enceinte d'émetteur (19) et un socle d'émetteur (21) relié à celle-ci, le socle d'émetteur (21) comportant au moins une ouverture pour gaz (23) qui est exécutée pour l'amenée d'un gaz de traitement dans une zone d'espace voisine de l'enceinte d'émetteur (19), **caractérisé en ce que** l'enceinte d'émetteur (19) est réalisée de manière à être plate et le socle d'émetteur (21) de manière à avoir la forme d'un cadre, le socle d'émetteur (21) faisant saillie sur le côté antérieur (22) de l'enceinte d'émetteur (19), côté qui émet le rayonnement lors du fonctionnement, et les côtés intérieurs saillants du socle d'émetteur (21) en forme de cadre étant pourvus de plusieurs ouvertures pour gaz (23).

2. Émetteur selon la revendication 1, les ouvertures pour gaz (23) étant disposées et orientées de manière à ce que le gaz de traitement puisse s'écouler dans une zone d'espace voisine de l'enceinte d'émetteur (19).

3. Émetteur selon la revendication 1 ou 2, l'enceinte d'émetteur étant pourvue d'au moins une ouverture de passage similaire à un tunnel qui est prévue pour l'amenée ou l'évacuation du gaz de traitement.

4. Émetteur selon l'une des revendications précédentes, comprenant un raccord de gaz (25) pour l'alimentation en gaz de traitement.

5. Émetteur selon l'une des revendications précédentes, le socle d'émetteur comportant au moins une ouverture pour gaz qui est exécutée pour l'évacuation d'un gaz de traitement.

6. Émetteur selon l'une des revendications précédentes, l'enceinte d'émetteur (19) étant formée, au moins par endroits, d'un matériau transparent au rayonnement.

7. Émetteur selon l'une des revendications précédentes, qui est étudié pour l'émission d'un rayonnement UV.

8. Émetteur selon l'une des revendications précédentes, qui est étudié pour une décharge à barrière diélectrique à l'intérieur de l'enceinte d'émetteur (19).

9. Utilisation d'un émetteur selon l'une des revendications précédentes pour la désinfection de surfaces planes ou courbes.
